# EUROPEAN PATENT APPLICATION

(11) **EP 2 481 383 A2**
(43) Date of publication of application: **01.08.2012**
(21) Application number: 11196251.0
(22) Date of filing: 30.12.2011
(51) Int. Cl.: A61F 13/15

(54) **Method of creating a cross-machine direction fold line boundary**

(30) Priority: 30.12.2010 US 201013428678
(71) Applicant: Curt G. Joa, Inc., Sheboygan Falls, Wisconsin 53085 (US)
(72) Inventor: Andrews, Robert E., Sheboygan, WI Wisconsin 53083 (US)
(74) Representative: Dee, Ian Mark

(57) **Abstract**

Apparatus and methods are provided to allow for creation of a cross-machine direction fold boundary, so that a laminate with a defined foldover can be created. A laid open two-ply material can then undergo processing operations such as addition of discrete disposable product components, or printing, and then be laid closed and sealed to form disposable products.

## Description

### Related Application

This application claims the benefit of copending U.S. Provisional Application Serial No. 61/428,678, filed 30 December 2010.

### Background of the Invention

This invention relates to an apparatus and method for creating a cross-machine direction fold line boundary that can be used in production of disposable absorbent articles.

### Summary of the Invention

In order to present a two-ply material with the plies spread open, two or more layers of material can be laid on one another and bonded to one another intermittently in the machine direction, at a relatively short cross-machine direction dimension. The top (or bottom) layers can then be folded over to present a laid-open type presentation so that material can be sandwiched between the plies, or other processing operations could be performed on the interior of the plies. In this manner, the bonding (either intermittent in the machine direction, or continuous down the length of the web in the machine direction) of the plies creates a boundary in the cross-machine direction, past which the fold cannot proceed in the cross-machine direction.

### Brief Description of the Drawings

Fig. 1 is a side view schematic of a representative web processing system;
Fig. 2A is a perspective view of a two ply material advancing in the machine direction, with intermittent bonds extending partially across the plies in the cross-machine direction;
Fig. 2B is a cross-sectional view of a two ply material intermittently bonded;
Fig. 3A is a perspective view of a two ply material advancing in the machine direction, with intermittent bonds extending partially across the plies in the cross-machine direction, the top ply having been folded over in the cross-machine direction to the intermittent bonds, the intermittent bonds creating a boundary which the foldover cannot go past;
Fig. 3B is a cross-sectional view of the product shown in Fig. 3A;
Fig. 4A is a perspective view of a two ply material advancing in the machine direction, with intermittent bonds extending partially across the plies in the cross-machine direction, as well as coterminous bonds extending widthwise in the cross machine direction;
Fig. 4B is a cross-sectional view of the product shown in Fig. 4A.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention, which is defined by the claims.

It is noted that the present techniques and apparatus are described herein with respect to products such as diapers, but as previously mentioned, can be applied to a wide variety of processes in which discrete components are applied sequentially.

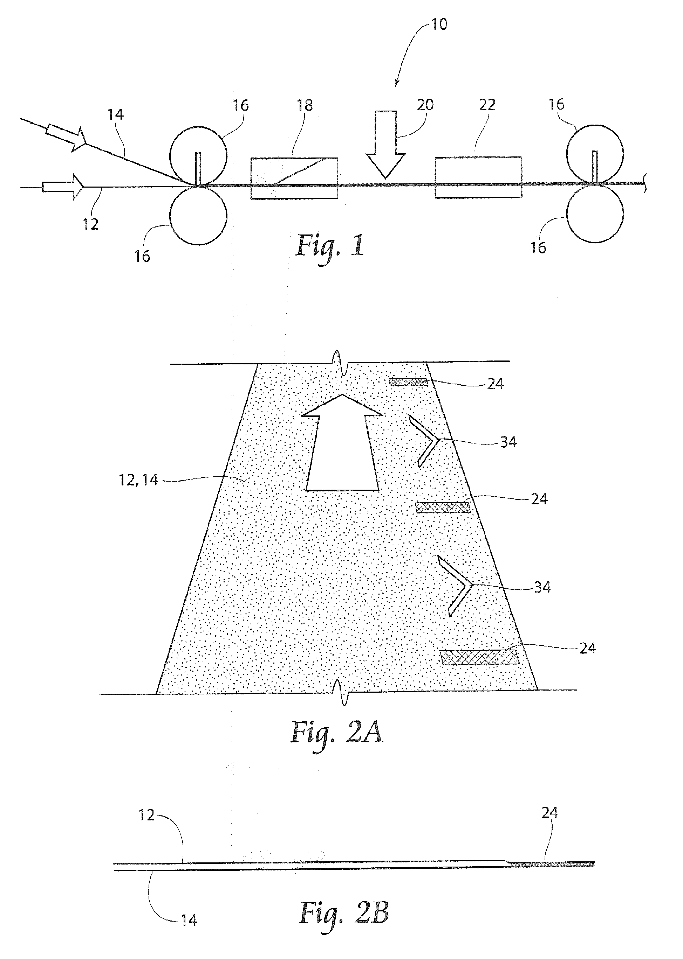

Referring now to Fig. 1, a side view schematic of a representative web processing system 10 is shown. Two plies of material 12 and 14 are laid down and pass through a bonding unit 16 to create intermittent bond sites 24, and optionally, patterned bond sites 34 (see Fig. 2A). The bonding unit 16 can be configured to create both the bond sites 24 and 34, or an additional bonding unit 16 can be employed to create the bond sites 34 (not shown).

In an optional embodiment, the intermittent bond sites 24 can instead be a continuous bond 24 running the length of the web in the machine direction (not shown).

Plies of material 12 and 14 can be non-woven, woven, elastic or inelastic etc. Generally, non-woven materials are preferred during some applications in diaper making. The bonding unit 16 can be and is preferred to be an ultrasonic bonding unit (horn/anvil) but of course any type of bond can be used to create the cross-machine direction fold boundary of the present invention, created by the intermittent bond sites 24.

After passing through the bonding unit 16, the two plies 12 and 14 pass through a unit 18, such as a folding or unfolding plow (such as those disclosed in U.S. Patent Nos. 4,171,239 or 7,374,627, incorporated herein by reference) to overturn one of the layers 12 or 14. Next, as will be described later, a processing operation 20 can be performed on the laid open two ply laminate, the laid-over or laid-open ply can then be re-folded back to its original laid closed orientation, and an additional bonding unity 16 can be used to further and additionally bond the plies 12 and 14 as desired.

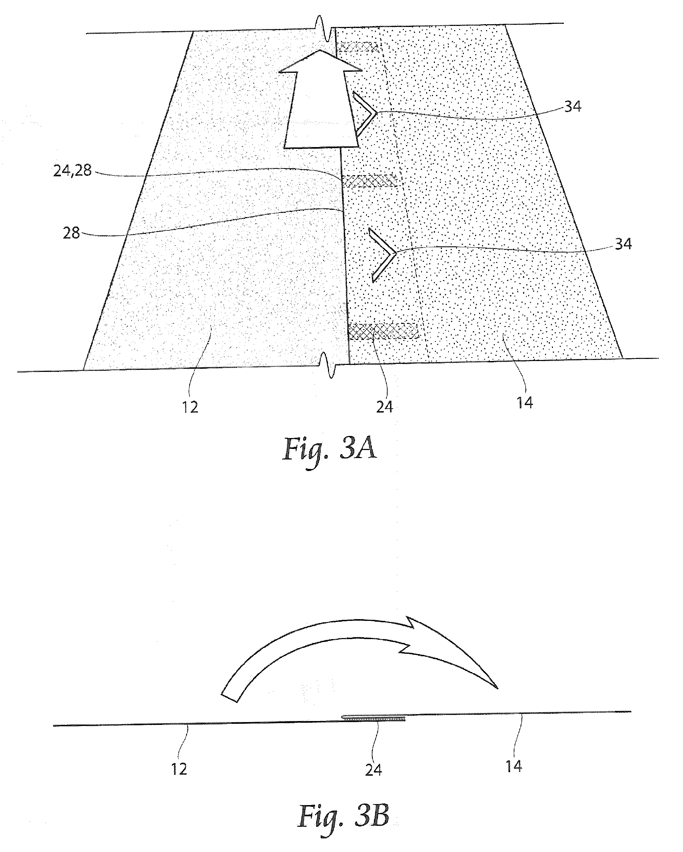

Referring now to Fig. 2A, the first steps of the processing operation are shown in a perspective view of a two ply material advancing in the machine direction. As can be seen, plies 12 and 14 have been bonded intermittently in the machine direction at intermittent bond sites 24, which define the cross-machine direction boundary at which the fold 28 (see Fig. 3A) will be effective upon the ply 14. It is preferred that bond sites 24 are spaced apart in the machine direction, preferably at a distance of some multiple of the product pitch, and that the bond sites 24 extend from relatively close to an edge of the plies 12 and 14 in the cross-machine direction.

The width of the bond sites 24 in the cross-machine direction is according to user preference and the design of the disposable product for which the plied material will eventually be used with. However, it is preferred that the width of the bond sites be coextensive with how wide the fold-over (described later) is desired.

The optional patterned bond sites 34 (shown in an exemplary embodiment as chevron-shaped) can be positioned either co-extensive in the cross-machine direction with the fold line 28 (not shown), or positioned closer to the non-folded edge of the plies 12 and 14 as shown in Figs. 2A and 3A.

Referring to Fig. 2B, a cross-sectional view of a two ply material intermittently bonded is shown. This orientation, with plies 12 and 14 in their original orientation, is considered a "closed" orientation.

Referring now to Fig. 3A, a perspective view of the two ply 12/14 material advancing in the machine direction, with intermittent bonds 24 extending partially across the plies 12/14 in the cross-machine direction is shown in an "open" or "laid open" orientation. The top ply 14 has been folded over in the cross-machine direction by folding plow 18 (see Fig. 18) to the intermittent bonds 24 to fold line 28, which is coextensive in the cross machine direction to the edge of the bond site 24 farthest from the edge of the web 10. In the cross machine direction, the intermittent bonds 24 effectively created a boundary or fold line 28 which the foldover cannot go past.

Fig. 3B is a cross-sectional view of the product shown in Fig. 3A in the laid open orientation. With the interior of the two ply 12/14 laminate having been exposed, processing operations can be performed on the interior by processors 20 (Fig. 1). For instance, if certain discrete portions of a disposable product are desired to be introduced in sandwich fashion, the discrete elements can be added to the interior of the two plies at this point. The discrete elements (not shown) can be absorbent cores, laminates, additives. Alternatively, certain operations such as scent additive or printing can be performed on the interiors of plies 12/14 in this laid open configuration.

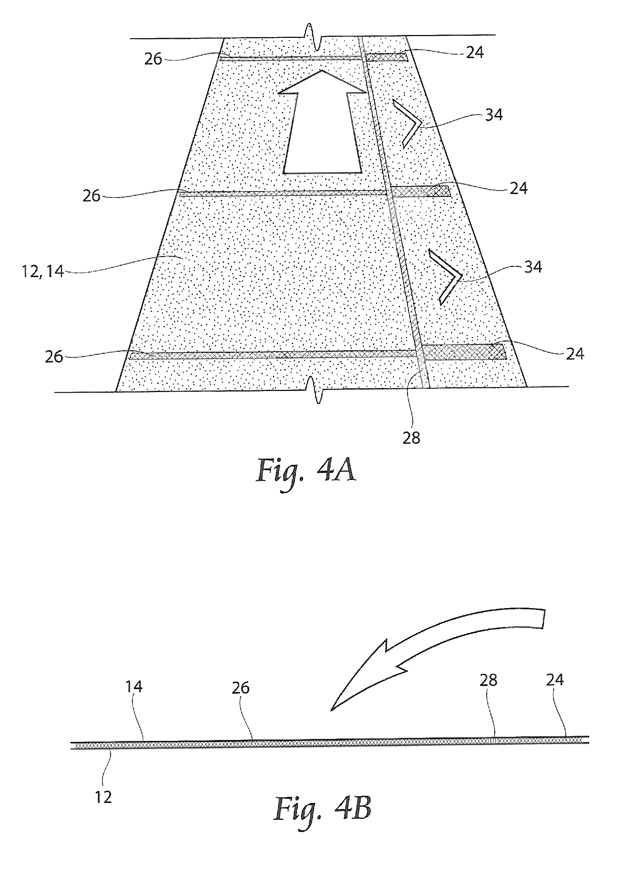

Referring now to Fig. 4A, the next step is to re-fold the top ply 14 back to its closed orientation, by use of folding plow 22 (Fig. 1). A perspective view of the two ply material advancing in the machine direction, with intermittent bonds 24 extending partially across the plies 12/14 in the cross-machine direction is shown. After the processing operations 20 have been performed and the plies 12/14 are returned to their closed orientation, it is preferred to pass the material through an additional intermittent bonding unit 16 to create coterminous bonds 26 extending generally in the width of the web 10 in the cross-machine direction. These coterminous bonds 26 extending widthwise in the cross machine direction at a preferred spacing to match intermittent bonds 24 and at a spacing of one or more bonds per product in the machine direction, in order to separate products. The closed orientation of the two ply material is shown in Fig. 4B, with the laminate returned to its closed orientation.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A method of creating a machine direction fold line comprising:
supplying a first and a second web both running in a machine direction;
creating a first bond pattern between said first and said second webs for a first distance in a cross-machine direction, said first distance less than a width of said first and second webs and defining a fold line boundary;
folding over said second web to create a machine direction fold line coextensive in the cross-machine direction with said fold line boundary.

2. A method according to claim 1, wherein said first bond pattern is intermittently applied in the machine direction as said webs run.

3. A method according to claim 1, wherein said first bond pattern is continuously applied in the machine direction as said webs run.

4. A method according to claim 1, the method further comprising creating a second bond pattern between said first and said second webs.

5. A method according to claim 4, said second bond pattern coextensive in the cross-machine direction with said fold line boundary.

6. A method according to claim 4, said second bond pattern not coextensive in the cross-machine direction with said fold line boundary.
